# EUROPEAN PATENT APPLICATION

(11) **EP 3 711 657 A1**
(43) Date of publication of application: **23.09.2020**
(21) Application number: 19164255.2
(22) Date of filing: 21.03.2019
(51) Int. Cl.: A61B 5/00, A61B 5/11

(54) **WALKING AID RECOMMENDATIONS**

(71) Applicant: Koninklijke Philips N.V., 5656 AG Eindhoven (NL)
(72) Inventor: EJUPI, Andreas, 5656 AE Eindhoven (NL); MOLLUS, Sabine, 5656 AE Eindhoven (NL); ANNEGARN, Janneke, 5656 AE Eindhoven (NL)
(74) Representative: de Haan, Poul Erik

(57) **Abstract**

Presented are concepts for recommending a walking aid for a subject. One such concept employs determining a characteristic of walking activity of the subject based on activity data representative of walking activity of the subject. A walking aid recommendation for the subject is then determines based on the characteristic of walking of the subject.

## Description

### FIELD OF THE INVENTION

This invention relates generally to providing walking aid recommendations for a subject, such as a person or a patient.

### BACKGROUND OF THE INVENTION

Walking aids (sometimes otherwise referred to as walkers or walking frames) are tools for people who need additional support to maintain balance or stability while walking. For example, a walking aid will typically improve a user's gait performance by increasing the base of support, provide weight-bearing support and increase the user's lateral stability. As a result, walking aids are widely used by injured, disabled, or older people.

There are currently many different types of walking aids available, including canes, four-post walkers, front-wheeled walkers, four-wheeled walkers and crutches.

Guidelines and recommendation about the prescription of walking aids are not well-established. Studies have shown that most subjects obtain their walking aid(s) device on their own or based on the advice of family or friends. Only about one-third of subjects obtained their device through a medical professional, and only 20 percent received education on how to use it. Also, studies have found that up to seventy percent of canes are faulty, damaged, or the wrong height.

As a result of the demands of walking aid use and inadequate/inappropriate walking aid selection, up to half of subjects stop using their walking aid soon after receiving it. Furthermore, improper fitting and selection of a walking aid can result in a poor gait pattern, which increases energy expenditure and a risk of falls.

Accordingly, there is a need to improve the process of recommending a walking aid for a subject.

### SUMMARY OF THE INVENTION

The invention aims to at least partly fulfil the aforementioned needs. To this end, the invention provides systems and methods as defined in the independent claims. The dependent claims provide advantageous embodiments.

There is provided a system for recommending a walking aid for a subject, wherein the system comprises:
a signal interface adapted to receive activity data representative of walking activity of the subject;
a processing unit adapted to determine a characteristic of walking activity of the subject based on the activity data, the characteristic of walking of the subject comprising information representing at least one of a pattern of walking of the subject and a parameter of walking of the subject; and
a recommendation unit adapted to determine a walking aid recommendation for the subject based on the characteristic of walking of the subject.

Proposed is the concept for recommending a walking aid for a subject based on a characteristic of walking of the subject. The characteristic of walking of the subject is derived from activity data representative of walking activity of the subject. By using activity data for the subject, one or more characteristics of walking specific to the subject may be identified. A recommendation based on the identified characteristic(s) may help to ensure the subject selects the most appropriate walking aid for their needs and/or walking activities. For example, a particular gait pattern may be detected based on activity data captured by sensors worn by a subject. The gait pattern may be best catered for by a particular gait pattern-optimised walking aid, and so, based on that gait pattern being detected for a subject, a recommendation that the subject uses the gait pattern-optimised walking aid may be generated.

Proposed embodiments may thus address a problem of identifying the correct (or most appropriate) timing to use a walking aid. Embodiments may also address the problem of selecting the optimal or most appropriate type of walking aid for a subject (e.g. by taking account of the subject's walking characteristic(s)).

Thus, there is proposed the concept of determining a characteristic of walking of the subject and determining a walking aid recommendation for the subject based on the characteristic of walking of the subject. It is proposed that a characteristic of how a subject walks, including aspects such as qualitative aspects and quantitative aspects may determine which walking aid is best suited to the subject. Exemplary qualitative aspects may include walking pattern, walking speed, walking intensity, gait pattern, walking stability, walking variability, and walking strength. Exemplary quantitative aspects may include number of walks (e.g. per day), typical walking duration (e.g. short vs. long), distance per walk, walking indoor vs. outdoor stair visits, and walking regularity. In addition, environmental factors, such as if a subject has to use stairs within a living environment, may also have an influence and thus be accounted for in generating a walking aid recommendation.

Existing sensors (e.g. as used for health monitoring), and particularly wearable sensors, provide opportunities to precisely capture and/or measure activity data that may be representative of walking activity of a subject. Using such data, one or more characteristics of walking activity of the subject may be determined, and this may contribute to selecting the correct type of walking aid. In particular, sensors may provide insights into activities of daily living (ADLs) and/or daily life routines, which may not be available in normal clinical practice.

Proposed embodiments may combine sensor technology with artificial intelligence to generate tailored, accurate and inexpensive walking aid recommendations, and this may be achieved on a relatively large-scale.

For instance, there may be proposed a system which includes wearable sensors and a cloud architecture to generate walking aids recommendations for subjects. Wearable sensors allow regular or continued monitoring of a subject's walking characteristic(s) (e.g. walking pattern or a parameter of walking). Information useful for determining a walking aid recommendation may therefore be automatically extracted from sensor signals and processed on one or more servers in the cloud. If such a system is used on a large scale (i.e. for multiple subjects), characteristics of walking for an individual subject can be captured and compared with a plurality of stored profiles. Recommendations may then be generated based on similarities between characteristics of walking for an individual subject and stored profiles. In this way, new subjects may be matched against existing profiles and the system may then provide one or more recommendations about walking aids to the subject. Such recommendation may, for example, detail which walking aid may be suited to the subject, based on information relating to what walking aid is used by the majority of other subjects with similar walking characteristics.

Embodiments may thus enable walking aid recommendations to be provided to one or more monitored subjects. For instance, accurate determination of a characteristic of walking of a subject may provide a better understanding of the walking aid requirements for the subject. Further, a forecast of the most appropriate walking aid may be provided based on the determined characteristic of walking of the subject (e.g. a prediction that a new type of walking aid will be needed may be provided in a timely manner). An expected time until walking aid requirements will alter (e.g. move to a next type of level of walking aid) may also be estimated by embodiments. Proposed embodiments may therefore enable improved (e.g. more accurate) forecasting of a subject's walking aid requirements.

Embodiments may therefore also enable the monitoring of a subject's health status by detecting and analysing irregularities or anomalies in a trend of a characteristic of walking of the subject. A trend of a characteristic of walking of the subject may be determined from input data relating to a plurality of event occurrences for the subject. For example, a characteristic of walking of a subject may comprise: walking speed; walking mobility; gait pattern; walking intensity; walking regularity; and gait abnormality. Embodiments may derive values of such health parameters from information representative of: walking activity of the subject that is provided for detected event occurrences.

An activity may comprise an Activity of Daily Living (ADL) that concerns a basic activity a subject may execute on a regular basis. Examples of such activities of daily living are drinking/eating; cooking; medicating; sleeping; toileting; bathing; washing, any kind of exercising such as walking, leisure activities such as reading or TV watching and many more etc. Thus, the invention may provide a way to monitor a characteristic of walking of a subject based on one or more ADLs (such as cooking, eating, exercising, opening doors, etc.) in a simple and easy to implement manner. Such a monitored characteristic may then be used to determine a walking aid recommendation for the subject.

In this regard, a health status of the subject may be described using one or more parameters for defining a state of physical or mental characteristics of a subject. Thus, reference to a subject's health status should not be taken to refer to an entire, overall or complete description of a subject's health (which may for example require a large number of health parameters and descriptors to be defined). Instead, reference to a subject's health status should be understood to refer to an indication of an aspect of the subject's health or well-being and which is described using a limited/restricted number of parameters (preferably fewer than ten in total, more preferably fewer than five in total, and even more preferably only one or two in total).

Also, a characteristic of walking of the subject may be described using one or more parameters for defining a state of physical or mental characteristics of a subject. Thus, reference to a subject's characteristic of walking should not be taken to refer to an entire, overall or complete description of a subject's walking (which may for example require a large number of parameters and descriptors to be defined). Instead, reference to a subject's characteristic of walking should be understood to refer to an indication of an aspect of the subject's walking activity and which may be described using a limited/restricted number of parameters, such as pattern of walking, walking speed; walking mobility; gait pattern; walking intensity; walking regularity; and gait abnormality (and preferably fewer than ten in total, more preferably fewer than five in total, and even more preferably only one or two in total).

The activity data representative of walking activity of the subject may comprise values at least one of: a velocity of movement of the subject; a distance travelled by a body part of the subject; a rate of acceleration of a body part of the subject; a location of the subject; and an air pressure incident on the subject. Air pressure data may, for example, provide information about height/altitude change of the subject (e.g. when walking stairs). Also, location data may provide information about whether the subject is walking indoors or outdoors for example. Such information may be obtained, for example, using simple and conventional sensors (such as a GPS sensor, a magnetometer, a heart rate sensor and/or an air pressure sensor for example) that are already widely employed in monitoring systems. Embodiments may therefore be simple and cheap to implement.

Some embodiments may comprise a sensor configured to be coupled to the subject and to generate activity data representative of walking activity of the subject, and preferably wherein the sensor comprises at least one of: an accelerometer; a gyroscope; a movement sensor; a pressure sensor; and a timing device. Such embodiments may therefore employ simple and conventional sensors that are already widely employed in monitoring systems, whilst also enabling the automatic and continued capture of activity data.

Embodiments may also comprise a user input interface adapted to receive a user input for defining or modifying input data. This has the advantage of enabling a user to manually provide and/or edit information (such as activity data representative of walking activity of the subject and/or a characteristic of walking of the subject), which may be useful in ensuring accurate and/or relevant information is employed for determinations.

A parameter of walking of the subject may comprise one or more qualitative aspects and/or quantitative of walking. Qualitative aspects of walking may, for example, include walking pattern, walking speed, walking intensity, gait pattern, walking stability, walking variability, and walking strength. Quantitative aspects of walking may, for example, include number of walks (e.g. per day), typical walking duration (e.g. short vs. long), distance per walk, walking indoor vs. outdoor (i.e. an indoors or outdoors status of the subject) stair visits, and walking regularity.

By way of example, the processing unit may be configured to process the activity data with a walking detection algorithm to detect walking activity. The processing algorithm may then be configured to determine a characteristic of walking of the subject based on detected walking activity. For instance, the walking detection algorithm may configured to detect peaks in a variation of a value with respect to time and to detect walking activity based on detected peaks. By way of example, United States Patent Application Publication Number US 2017/0000384 A1 presents a method of processing measurements of acceleration to identify steps by a user. Other such walking activity detection methods and/or algorithms are known and may be employed by embodiments. Since methods for detecting walking activity from sensor measurements are numerous and known, detailed description of such methods is hereby omitted.

By detecting values of a property of a subject and/or an object that the subject interacts with, a trend in the detected values over time may be identified and, from such a trend, a physical or mental capability of a subject may be monitored. For example, a trend of declining/decreasing velocity or speed of movement of the subject (as detected directly, using cameras, for example, or inferred using a accelerometer affixed to the subject, or as detected indirectly using a accelerometer integrated into an object that is moved by the subject) may be used to identify and monitor the subject's characteristic of walking.

Embodiments may be further adapted to store obtained data in a database adapted to store historical data relating to one or more previously obtained values or recommendations. Previously determined values of a parameter for the subject may therefore be stored, in a historical database for example, and then used in subsequent calculations. Furthermore, currently determined values may be used to re-calculate or refine a previously determined parameter value or trend.

In some embodiments, the recommendation unit may comprise: a classification unit configured to determine a classification of the subject based on the characteristic of walking activity of the subject; and an analysis unit configured to determine a walking aid recommendation for the subject based on the classification of the subject. The classification unit may be configured to process the characteristic of walking activity of the subject with at least one of: a machine learning algorithm; a neural network; rule-based algorithm; and comparison algorithm to determine a classification of the subject. Similarly, the analysis unit may be configured to process the classification of the subject with at least one of: a machine learning algorithm; a neural network; rule-based algorithm; and comparison algorithm to determine a walking aid recommendation for the subject. By way of example, the classification of the subject may be compared with a plurality of user profiles to identify a user profile which best matches the classification. The matched user profile may then be used to identify a walking aid recommendation, e.g. the user profile may have a specific walking aid associated with it.

When the characteristic of walking of the subject comprises information representing a pattern of walking of the subject, the classification unit may be configured to identify a trend in the pattern of walking of the subject and to determine a classification of the subject based on the identified trend.

Furthermore, the classification unit may be configured to detect one or more irregularities in the determined trend, and to determine a classification of the subject based on the detected one or more irregularities in the determined trend. For example, in some embodiment, the classification unit may be configured to identify whether there are one or more irregularities in the determined trend based on at least one of: a comparison of the determined trend with a threshold value; a comparison of a value of a characteristic of walking of the subject with an average value; and a comparison of the determined trend with a predetermined trend. Thus, to detect an irregularity, the classification unit may undertake a comparison of the determined trend with a threshold value. For example, the classification unit may employ a data processing unit that compares the determined trend with a threshold value. The threshold may be preprogramed and fixed, but it may be preferable to enable the threshold value to be set by a user preference. Also the threshold value may relate to a future value, and the trend may be extrapolated for comparison of an extrapolated value with the future threshold value. This may identify when a threshold value may be exceeded in the future, for example.

Also, the threshold value may be determined based on at least one of: one or more previously obtained values of a property of at least one of: a pattern of walking of the subject and a parameter of walking of the subject; and one or more previously determined changes in values of a property of at least one of: pattern of walking of the subject and a parameter of walking of the subject. The threshold may thus be based on previously detected values and/or previously determined event occurrences for the subject. In other words, the threshold may be defined by taking account of a history of detected values and/or a history of events so that it can be used to identify outlying values or anomalies.

A walking aid recommendation for the subject may, for example, comprise: a description of a walking aid; and a numerical value associated with the walking aid.

The recommendation unit may be further adapted to generate a control signal for modifying a graphical element based on the walking aid recommendation for the subject. Further, the system may further comprise a display system adapted to display the graphical element in accordance with the control signal generated by the recommendation unit. In this way, a user (such as a care giver) may have an appropriately arranged display system that can receive and display information about the walking status and/or health status of the subject, and that subject may be remotely located from the user. Embodiments may therefore enable a user to remotely monitor a subject (such a patient or elderly subject) using a portable display device, such as a laptop, tablet computer, mobile phone, PDA, etc.

It will be appreciated that all or part of the recommendation unit may comprise one or more data processing units. For example, the recommendation unit may be implemented using a single processor which is adapted to undertake data processing in order to determine a walking aid recommendation for the subject.

The monitor unit may be remotely located from one or more sensors that provide the activity data representative of walking activity of the subject, and a signal representative of the activity data may be communicated to the processing unit or the recommendation unit via a communication link.

The system may further comprise: a server device comprising the recommendation unit; and a client device comprising the signal interface. Dedicated data processing means may therefore be employed for the purpose of determining a walking aid recommendation for the subject, thus reducing processing requirements or capabilities of other components or devices of the system.

The system may further comprise a client device, wherein the client device comprises the recommendation unit and a display system. In other words, a user (such as a monitored subject or a care giver) may have an appropriately arranged client device (such as a laptop, tablet computer, mobile phone, PDA, etc.) which processes received data in order to determine a walking aid recommendation for the subject.

Thus, processing may be hosted at a different location from where the sensing or data provision happens. For example, for reasons of power efficiency (e.g. to improve battery lifetime) it might be advantageous to execute only part of the processing at a sensor location, thereby reducing associated costs, processing power, transmission requirements, etc.

Thus, it will be understood that processing capabilities may therefore be distributed throughout the system in different ways according to predetermined constraints and/or availability of processing resources.

Embodiments may comprise a sensor arrangement/system positioned in a strategic position so that it detects the appropriate values of walking activity without the subject needing to intentionally or consciously activate/operate the sensor. In this way, a subject may only need to undertake their normal activities. Such strategic positioning may ensure that a value of a property of the subject or environment can be automatically and accurately obtained, and this may not require the subject to remember to undertake any special or additional activities in order for a value to be detected by the sensor. This may remove the risk of the subject forgetting to activate a sensor (e.g. by pressing a button), for example.

There exist many sensors that can be employed by a monitoring system according to an embodiment. Typical sensors include PIR (Passive Infra-Red; measure movement and presence), OC (open-close; measure state of doors, in particular front doors, windows, and cupboards, including refrigerators), power sensors (measure current consumption of appliances, such as microwave, water cookers, TV, etc.); and pressure sensors or mats (measure occupancy of user sitting in chair, lying in bed, standing on door mat in front of front door, being at toilet, etc.). Many others exist and are conceivable, such as sensors to signal light switch state, or sensors that measure environmental conditions such as humidity, CO2 level (or CO and smoke), Particulate Matter level, etc. A further range of sensors are those based on physical quantities, such as accelerometers, magnetometers, gyroscopes, and air pressure sensors. Accelerometers, for example, can also measure state of doors and their open-close movements or measure speed or velocity of movement of a subject. Yet another range of sensors consists of microphones and cameras (including infra-red, or even UV and beyond, part of spectrum), to which also belong GPS and location-sensitive IR. Ultra-sound or RF-based sensors, including RFID tagging, provide additional input. Appliances having an own IP-address, known as the internet-of-things, provide further sensor input signals that can be taken by the smart-home system.

Although the sensor(s) may be mounted in the monitoring environment (e.g. the subject's home), they may also be attached to user utilities (such as a keyring) or put in clothes, in a pocket or bag, or as insole or undergarment, etc. They may also be fabricated to be worn explicitly like a wrist-watch or pendant. Further, the sensors may communicate their output signals via a wired or wireless connection, or a combination thereof. Accordingly, in an embodiment, a sensor may be adapted to be coupled to the subject or the object. The object may for example comprise an item of furniture (such as a fridge, cupboard, wardrobe, seat, door, white good, etc.) adapted to be used in the execution of an ADL.

Employed sensors may also be adapted to undertake primary processing of the detected values, such a signal filtering, sampling, conditioning, etc., so as to reduce required transmission bandwidth and/or transmission duration for example. Alternatively, the sensors can send raw data.

Non-intrusive monitoring of walking activity may therefore be realized with relatively simple sensors that provide data on specific ambient conditions or properties/parameters of an object, an environment (such as temperature or humidity for example), or properties of the subject (such as movement, weight, speed, weight, and/or distance travelled for example). Such sensors for measuring ambient condition or properties/parameters of the object or environment may be simple, small and/or cheap. Also, the movement of the subject may be detected with, for example, a Passive Infrared (PIR) sensor which is a cheap component. Movement sensors may be used to switch on lighting and people are therefore typically familiar with their usage.

Thus, systems of the invention may employ conventional sensors and/or existing sensor arrangements. Also, embodiments may employ sensors that are considered to be non-intrusive and more easily accepted by the monitored subject. Yet, with the data provided by these sensors, walking activities may be determined and provide information on the subject being monitored.

Such sensors may be employed by, or in conjunction with, embodiments so as to increase the number and/or accuracy of monitored activities. They may also be used to confirm or qualify readings taken by a sensor, so that spurious or unintentional measurements are avoided. For example, signals from a location sensor worn by the monitored subject may be used to confirm if readings taken by a sensing system are indeed attributable to the monitored subject or some other subject or animal (such as their pet), for example.

According to another aspect of the invention, there is provided a method for recommending a walking aid for a subject, wherein the system comprises:
obtaining activity data representative of walking activity of the subject;
determining a characteristic of walking of the subject based on the activity data, the characteristic of walking of the subject comprising information representing at least one of a pattern of walking of the subject and a parameter of walking of the subject; and
determining a walking aid recommendation for the subject based on the characteristic of walking of the subject.

There is also provided a computer program product for recommending a walking aid for a subject, wherein the computer program product comprises a computer-readable storage medium having computer-readable program code embodied therewith, the computer-readable program code configured to perform all of the steps of an embodiment.

A computer system may be provided which comprises: a computer program product according to an embodiment; and one or more processors adapted to perform a method according to an embodiment by execution of the computer-readable program code of said computer program product.

These and other aspects of the invention will be apparent from and elucidated with reference to the embodiment(s) described hereinafter.

### BRIEF DESCRIPTION OF THE DRAWINGS

Examples in accordance with aspects of the invention will now be described in detail with reference to the accompanying schematic drawings, in which:
Figure 1 is a simplified block diagram of a system for recommending a walking aid for a subject according to an embodiment;
Figure 2 depicts a flow diagram of a method for recommending a walking aid for a subject according to an exemplary embodiment;
Figure 3 is a simplified block diagram of a system according to an embodiment;
Figure 4 is illustrates a method according to an embodiment; and
Figure 5 is a simplified block diagram of a computer within which one or more parts of an embodiment may be employed.

### DETAILED DESCRIPTION OF THE EMBODIMENTS

Proposed is a concept for recommending a walking aid for a subject (such as a patient or subject) based on a characteristic of walking of the subject that is determined using activity data representative of walking activity of the subject. This may be useful for the purpose of unobtrusively and/or automatically suggesting an optimal walking aid for a subject based on his/her unique walking trait(s). Such subjects may, for instance, include a disabled subject, an elderly subject, an injured subject, a medical patient, etc. Elderly subjects can mean subjects above 65 years, above 70, or above 80 years old.

Illustrative embodiments may be utilized in many different types of monitoring environments, such as a hospital, ward, care home, subject's home, etc. In order to provide a context for the description of elements and functionality of the illustrative embodiments, the Figures are provided hereafter as examples of how aspects of the illustrative embodiments may be implemented. It should therefore be appreciated the Figures are only examples and are not intended to assert or imply any limitation with regard to the environments, systems or methods in which aspects or embodiments of the present invention may be implemented.

In general, to be able to observe or identify daily behavior or activities of a subject one may monitor ADLs of a subject. From such monitoring, one may obtain activity data representative of walking activity of the subject. From such data, one or more characteristics of walking of the subject may be inferred or calculated. For instance, a measure of walking capability may be determined.

A large class of physical or mental capabilities can relate to an ADL routine of the subject. For example, a physical capability may be inferred from the speed by which a subject moves between locations and/or the number of times a subject negotiates a set of stairs.

Embodiments of the present invention are directed toward enabling information about a subject's walking (e.g. physical capability) to be obtained and potentially monitored. Using this information, a walking aid recommendation may be determined. Such information may also be useful for monitoring the health or well-being of a subject. Further, it may also be useful for predicting when walking aid requirements of the subject may change (e.g. increase) so as to require a different type of walking aid.

A characteristic of walking of the subject may, for example, be determined using a single sensor (e.g. an accelerometer) installed at a single location (e.g. on the user), thus reducing the burden, cost and/or complexity of a system according to an embodiment. It may also help to ensure that walking activity of a subject is accurately detected, thus improving the accuracy of recommendations. Such a proposed concept for recommending a walking aid for a subject may therefore be employed in a system for monitoring ADLs of a subject within an environment.

Physical activities of a subject may be detected or inferred from sensor output signals and there already exist systems and methods for such detection or inference. Accordingly, the proposed concepts may be used in conjunction with existing ADL detection or monitoring systems/methods. For example, Dries Vermeiren et al describe a system based on 2 tri-axial accelerometers to detect the ADLs of a patient in a paper entitled "Detecting Human Motion: Introducing Step, Fall and ADL algorithms". Also, H Pirsiavas et al describe algorithms for detecting ADLs in first-subject camera views in paper entitled "Detecting activities of daily living in first-subject camera views" (CVPR, 2012). United States Patent Application Publication Number US 2017/0000384 A1 presents a method of processing measurements of acceleration to identify steps by a user. Because many such detection or monitoring methods/systems are known and any one or more of these may be employed, detailed description of such methods/systems is omitted from this description.

Embodiments also employ the concept that a characteristic of walking of a subject may be determined based on activity data representative of walking activity of the subject. By using a characteristic of a subject's walking, a preferred or optimal walking aid for a subject may be identified and recommended for use by the subject. For example, by identifying a gait pattern of the subject from accelerometer data (generated by an accelerometer worn by the subject), a specific walking aid designed for the gait pattern may be determined. Based on this, the walking aid may be recommended to the subject. By way of further example, if a detected walking speed is below a predetermined threshold, a wheeled walking aid may be identified as optimal and recommended.

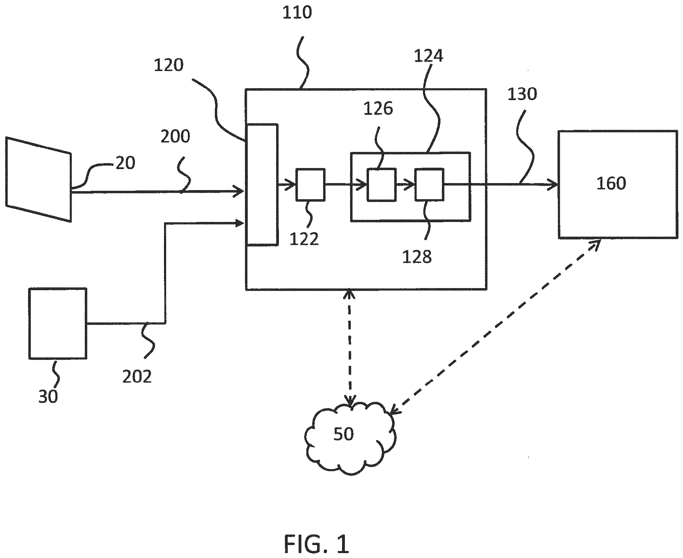

Fig. 1 shows an embodiment of a system according to the invention comprising an accelerometer 20 configured to be worn by the subject. In this way, the subject need only undertake their normal activities and may not even be aware that they are being monitored. Such positioning (worn on an ankle of the subject, for example) may ensure that a property of the subject's walking activity (such as: a velocity or speed of movement of subject's foot/ankle; a distance travelled by the subject; a rate of acceleration of the subject's ankle/foot, for example) can be automatically and accurately obtained without requiring the subject to remember to undertake any special or additional activities in order for walking activity of the subject to be detected. For example, it can remove the need for a subject to perform a specific additional action (e.g. pressing a button) in order to activate the accelerometer 20.

The accelerometer 20 comprises a motion sensing arrangement that is adapted to determine a velocity of movement of subject's ankle, and a distance travelled by the subject. The accelerometer 20 may obtain numerous measurements before, during and after the subject undertakes walking activity.

The accelerometer 20 is adapted to output sensor output signals 200 which are representative of the detected value(s) of the walking activity. Of course, many more sensors may be employed so as to provide signals indicative of detected values of properties of the subject. For example, the movement of the subject between locations may be detected using one or more presence detectors. Such additional signals may be useful for identifying which of the sensor output signals 200 are indicative of a walking activity. They may also be used to confirm or qualify values detected by the accelerometer 20, so that spurious or unintentional measurements are avoided. For example, signals from a location sensor worn by the monitored subject may be used to confirm if values detected by the accelerometer 20 are indeed attributable to the monitored subject walking between locations, for example.

The system also comprise an input module 30 adapted to receive user inputs indicative of walking activity provided to the subject. Here, the input module 30 comprises a tablet computer that is carried by the subject's carer or medial assistant and includes an application for facilitating the provision of information regarding walking activities undertaken by the subject. In this way, the system provides for user input of activity data comprising information representative of walking activity of the subject. The subject's carer or medial assistant need only undertake their normal care activities when caring for the subject and then use the application to provide information describing the observed walking activity.

The input module 30 is adapted to output walking activity signals 202 which are representative of the observed walking activity of the subject.

The accelerometer 20 and input module 30 both communicate their output signals 200, 202 via a respective wired or wireless connection. By way of example, the wireless connection may comprise a short-to-medium-range communication link. For the avoidance of doubt, short-to-medium-range communication link may be taken to mean a short-range or medium-range communication link having a range of up to around 100 meters. In short-range communication links designed for very short communication distances, signals typically travel from a few centimetres to several metres, whereas, in medium-range communication links designed for short to medium communication distances, signals typically travel up to 100 meters. Examples of short-range wireless communication links are ANT+, Bluetooth, Bluetooth low energy, IEEE 802.15.4, ISA100a, Infrared (IrDA), , Near Field Communication (NFC), RFID, 6LoWPAN, UWB, Wireless HART, Wireless HD, Wireless USB, ZigBee. Examples of medium-range communication links include Wi-Fi, ISM Band, Z-Wave. Here, the output signals are not encrypted for communication via the wired or wireless connection in a secured manner. However, it will be appreciated that, in other embodiment, one or more encryption techniques and/or one or more secure communication links may be employed for the communication of signals in the system.

The system further comprises a system 110 for recommending a walking aid for a subject according to an embodiment. The system comprises a signal interface 120 adapted to receive the sensor output signals 200 and the walking activity signals 202. The signal interface 120 provide the received signals 200,202 to a processing unit 122 of the system 110.

The processing unit 122 adapted to determine a characteristic of walking of the subject based on the activity data based on the received signals 200,202. For this purpose, the processing unit 122 may communicate with one or more data processing resources available in the internet or "cloud" 50. Such data processing resources may undertake part or all of the processing required to infer or determine a characteristic of walking of the subject based on the activity data based on the received signals 200, 202. Thus, the embodiment may employ distributed processing principles.

The processing unit 122 is adapted to provide information about the determined a characteristic of walking of the subject to a recommendation unit 124 of the system 110. The recommendation unit 124 is adapted to determine a walking aid recommendation for the subject based on the characteristic of walking of the subject. Again, for this purpose, the recommendation unit 124 may communicate with one or more data processing resources available in the internet or "cloud" 50. Such data processing resources may undertake part or all of the processing required to determine a walking aid recommendation for the subject.

More specifically, in this embodiment the recommendation unit 124 comprises a classification unit 126 configured to determine a classification of the subject based on the characteristic of walking activity of the subject., The recommendation unit 124 also comprises an analysis unit 128 configured to determine a walking aid recommendation for the subject based on the classification of the subject.

In more detail, the analysis unit 128 is configured to process the classification of the subject with at least one of: a machine learning algorithm; a neural network; rule-based algorithm; and comparison algorithm to determine a walking aid recommendation for the subject. As above, the analysis unit 128 may communicate with one or more data processing resources available in the internet or "cloud" 50. Such data processing resources may undertake part or all of the processing required to determine a walking aid recommendation.

Also, where the characteristic of walking of the subject comprises information representing a pattern of walking of the subject, the classification unit 126 is configured to identify a trend in the pattern of walking of the subject and to determine a classification of the subject based on the identified trend. In this example, the classification unit 126 is configured to detect one or more irregularities in the determined trend, and to determine a classification of the subject based on the detected one or more irregularities in the determined trend. For instance, the classification unit 126 is configured to identify whether there are one or more irregularities in the determined trend based on at least one of: a comparison of the determined trend with a threshold value; a comparison of a value of a characteristic of walking of the subject with an average value; and a comparison of the determined trend with a predetermined trend.

Accordingly, based on detected irregularities and their respective classification (i.e. type), the recommendation unit 124 determines a recommendation of a preferred or optimal walking aid for the subject. For example, the walking aid recommendation for the subject may comprises: a description of a walking aid; and a numerical value (e.g. score or suitability percentage) associated with the walking aid.

The recommendation unit 124 is further adapted to generate an output signal 130 representative of the determined walking aid recommendation for the subject. In other words, after having analysed the characteristic(s) of walking of the subject (derived from input representative of walking activity of the subject), an output signal 130 representative of a determined walking aid recommendation for the subject is generated.

The system further comprises a graphical user interface (GUI) 160 for providing information to one or more users. The output signal 130 is provided to the GUI 160 via wired or wireless connection. By way of example, the wireless connection may comprise a short-to-medium-range communicate on link. As indicated in Figure 1, the output signal 130 is provided to the GUI 160 from system 110. However, where the system, has made use of data processing resources via the internet or cloud 50), an output signal may be made available to the GUI 160 via the internet or cloud 50.

Based on the output signal 130, the GUI 160 is adapted to communicate information by displaying one or more graphical elements in a display area of the GUI 160. In this way, the system may communicate information about walking aid recommendation for the monitored subject. For example, the GUI 160 may be used to display graphical elements to a medical practitioner, a caregiver, a family member or close relative. Alternatively, or in addition, the GUI 160 may be adapted to display graphical elements to the monitored subject.

Although the embodiment described above employs an accelerometer to detect walking activity of a monitored subject, it will be understood that many other different properties of a subject and/or objects manipulated by the subject may be detected using sensors (of the same or different type) in alternative embodiments. For example, other embodiments may be arranged to: detect a velocity or speed of movement of the subject or an object; detect a a distance travelled by the object or a body part of the subject; detect a rate of acceleration of the object or body part of the subject; and/or detect a reaction time of the subject.

By way of further example, stair usage/behaviour, and the other similar walking-based ADLs, can be employed. Stair traversing speed may be described using a begin and end time of stair usage, and these times (as well as their duration) can form a statistic that can be monitored over time to vary within bounds. Such bounds can be set manually, but, for example, also determined as once or twice the standard deviation in the values of the statistic (e.g. stair traversal duration) over a predetermined time period (e.g. the past month).

Other ADL behaviours can be converted into a statistic that is representative of walking activity of the subject and monitored in a comparable manner. Examples include, time/duration walking between rooms, number of stair visits per day, leaving house, etc.

It will be appreciated that other embodiments may provide a system that employs wearable sensors and a cloud architecture. The wearable sensors allow automatic and/or regular monitoring of a subject's walking activity, and may also capture environmental factors indicative of walking activity (e.g. detect if stairs are avoided or used infrequently). Data regarding waking activity may be automatically extracted from sensor signals and processed on a server in the cloud.

When used on a large scale, embodiments may identify characteristics of walking of multiple subjects and walking aid recommendations may be made based on similarities between subject profiles. Further, new users may be matched against existing profiles and the recommendation unit may then provide walking aid recommendations based on what walking aid is used by the majority of other users with similar walking characteristics (e.g. similar profiles).

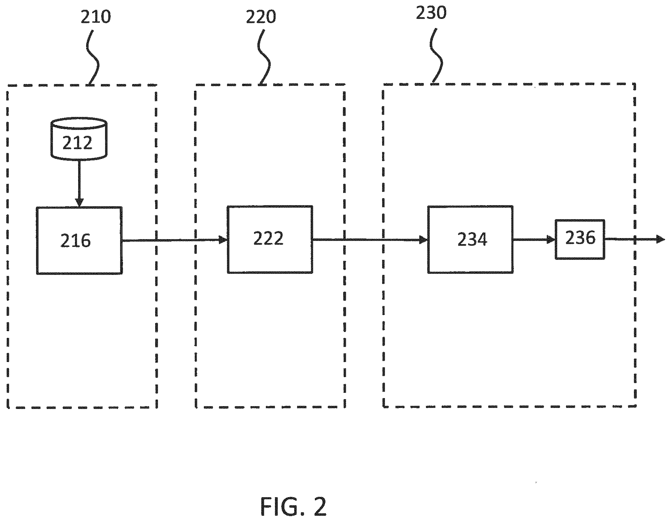

Referring now to Figure 2, there is depicted a flow diagram of a method for recommending a walking aid for a subject according to an exemplary embodiment.

In step 210, a proposed system receives input data. Here, the input data comprises: (i) sensor data 212 comprising information representative of walking activity of the subject.

By way of example, the sensor data 212 may be provided from activity sensors that are mounted in the subject's premises. Such sensors may be adapted to monitor activity and/or behaviour (e.g. walking speed, activity level, sleeping time, wake-up time) of the subject. The sensor data may, for instance, comprise values of: a velocity of movement of the subject; a distance travelled by a body part of the subject; a rate of acceleration of a body part of the subject; a location of the subject; and an air pressure incident on the subject.

The received data 212 is collated in time series 216 of data points which is then provided to the next step of walking analysis 220. In the step 220 of trend analysis, a characteristic 222 of walking of the subject is determined based on the received and collated input data 216. The characteristic of walking of the subject can, for example, comprise information representing at least one of a pattern of walking of the subject and a parameter of walking of the subject. By way of example, a parameter of walking of the subject in this embodiment comprises at least one of: a walking speed of the subject; a measure of walking mobility of the subject; a gait pattern of the subject; a walking intensity of the subject; a walking regularity of the subject; a number of stair visits of the subject; and a gait abnormality of the subject.

Next, in step 230, the characteristic(s) of walking of the subject is/are analysed to determine a classification 234 of the subject. A walking aid recommendation 236 for the subject is then determined based on the classification of the subject. Put another way, based on the input data, the system categorises walking activity of the subject and uses the categorisation of the subject to determined a walking aid recommendation for the subject.

As part of 230, the method processes the classification 234 of the subject with at least one of: a machine learning algorithm; a neural network; rule-based algorithm; and comparison algorithm to determine a walking aid recommendation for the subject. For instance the classification 234 may be compared against a plurality of user profiles to identify the profile having the closest match. From the matching user profile, a walking aid which is associated with that user profile (e.g. because it is the best or optimal walking aid for persons having the user profile) can then be identified. In other words, different user profiles may be associated with different, preferred or optimal walking aids, and a walking aid for the subject may be identified by determining a user profile which most closely matches the classification 234.

By detecting walking activity of a subject, characteristics of walking of the subject may be identified and, from such characteristics, an optimal or preferred walking aid may be determined. For example, an irregular gait pattern may be identified from a comparison of data from movement sensors worn on either leg of a monitored subject. It may then be determined (e.g. by comparison with one or more lookup tables or predefined user profiles) that the irregular gait pattern is best suited to a particular walking aid (e.g. that is specifically designed to cater for the gait pattern).

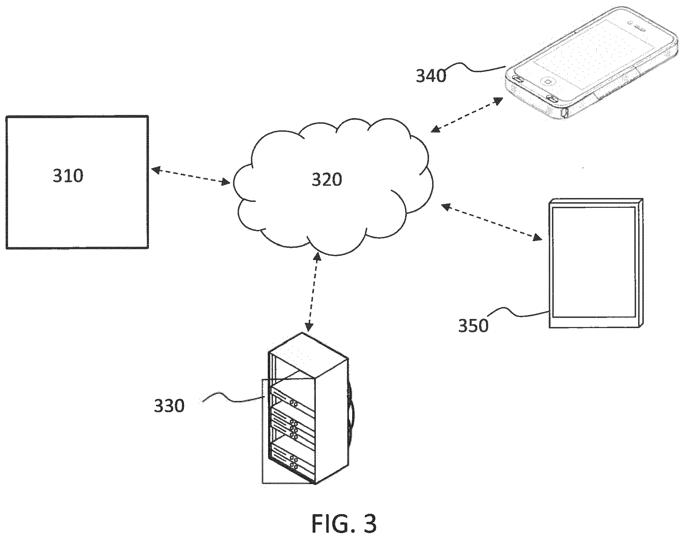

Referring now to Fig. 3, there is depicted another embodiment of a system according to the invention comprising a walking activity sensing system 310 adapted to detect walking activity of the subject. Here, the walking activity sensing system 310 comprises a high-resolution accelerometer system adapted to be integrated into a device carried or worn by the subject. The walking activity sensing system 310 is adapted to output one or more signals which are representative of the detected value(s) of a subject's movements.

Although this embodiment has been described as integrating the walking activity sensing system 310 into a portable device, it will be understood that, in alternative embodiments, the activity sensing system 310 may be provided separately from the subject such that it is installed throughout a monitoring environment for example. Such an alternative arrangement may avoid the need for the subject to carry or wear a device, and thus be less intrusive and/or cheaper. For example, instead of being integrated into a small portable device, the activity sensing system may comprises movement sensors (like Passive Infra-Red sensors and Open-Close sensors) positioned in various rooms so as to detect the subject's presence upon entering and leaving the various rooms.

The walking activity sensing system 310 communicates the output signals via the internet 320 (using a wired or wireless connection for example) to a remotely located data processing system 330 (such as server). Here, the data processing system comprises a system for recommending a walking aid for a subject.

The data processing system 330 is adapted to receive the one or more output signals from the walking activity sensing system 310 and process, using a processing unit (not shown), the received signal(s) in accordance with an inference/detection algorithm in order to infer/determine a characteristic of walking of the subject based on the activity data. More specifically, the algorithm processes the received sensor output signal(s) in combination with a walking detection algorithm which is configured to detect peaks in a variation of a value with respect to time and to detect walking activity based on detected peaks. Of course, it will be understood that other algorithms and/or techniques for identifying a characteristic of walking may be used, and the trend processing unit may use any of such method (or combination thereof).

Information about a determined characteristic of walking of the subject is passed to a recommendation unit (not shown) of the data processing system 300. The recommendation unit is adapted to a walking aid recommendation for the subject based on the characteristic of walking of the subject. More specifically, the recommendation unit classifies the subject based on the characteristic of walking activity of the subject, and then determines a walking aid recommendation for the subject based on the classification of the subject.

Thus, the data processing system 330 provides a centrally accessible processing resource that can receive information from the walking activity sensing system and run one or more algorithms to transform the received information into a walking aid recommendation for the subject. Information relating to the walking activity, characteristic(s) of walking, and/or walking aid recommendation can be stored by the data processing unit (for example, in a database) and provided to other components of the system. Such provision of information about walking activity, characteristic(s) of walking, and/or walking aid recommendation may be undertaken in response to a receiving a request (via the internet 320 for example) and/or may be undertaken without request (i.e. 'pushed').

For the purpose of receiving information about a walking aid recommendation from the data processing system 330, and thus to enable the subject's walking aid recommendations to conveyed and monitored, the system further comprises first 340 and second 350 mobile computing devices.

Here, the first mobile computing device 340 is a mobile telephone device (such as a smartphone) with a display for displaying graphical elements representative of a subject's walking aid recommendation. The second mobile computing device 350 is a mobile computer such as a Laptop or Tablet computer with a display for displaying graphical elements representative of a subject's walking activity, characteristic(s) of walking, and walking aid recommendation(s).

The data processing system 330 is adapted to communicate output signals to the first 340 and second 350 mobile computing devices via the internet 320 (using a wired or wireless connection for example). As mentioned above, this may be undertaken in response to receiving a request from the first 340 or second 350 mobile computing devices.

Based on the received output signals, the first 340 and second 350 mobile computing devices are adapted to display one or more graphical elements in a display area provided by their respective display. For this purpose, the first 340 and second 350 mobile computing devices each comprise a software application for processing, decrypting and/or interpreting received output signals in order to determine how to display graphical elements. Thus, the first 340 and second 350 mobile computing devices each comprise a processing arrangement adapted to determine one or more values representative of a walking aid recommendation, and to generate a display control signal for modifying at least one of the size, shape, position, orientation, pulsation or colour of the graphical element based on the one or more values representative of a walking aid recommendation.

The system can therefore communicate information about a walking aid recommendation to users of the first 340 and second 350 mobile computing devices, respectively. Further, the second mobile computing device 350 may communicate information about a subject's walking activity and, characteristic(s) of walking, in addition to the walking aid recommendation. For example, each of the first 340 and second 350 mobile computing devices may be used to display graphical elements to a medical practitioner, a caregiver, a family member or close relative.

Implementations of the system of Figure 3 may vary between: (i) a situation where the data processing system 330 communicates display-ready data, which may for example comprise display data including graphical elements (e.g. in JPEG or other image formats) that are simply displayed to a user of a mobile computing device using conventional image or webpage display (which can be web based browser etc.); to (ii) a situation where the data processing system 330 communicates raw activity data that the receiving mobile computing device then processes to determine a characteristic of walking of the subject based on the activity data and then determines a walking aid recommendation for the subject based on the characteristic of walking of the subject (for example, using local software running on the mobile computing device).

Of course, in other implementations, the processing may be shared between the data processing system 330 and a receiving mobile computing device such that part of the data generated at data processing system 330 is sent to the mobile computing device for further processing by local dedicated software of the mobile computing device. Embodiments may therefore employ server-side processing, client-side processing, or any combination thereof.

Further, where the data processing system 330 does not 'push' information (e.g. output signals), but rather communicates information in response to receiving a request, the user of a device making such a request may be required to confirm or authenticate their identity and/or security credentials in order for the information to be communicated.

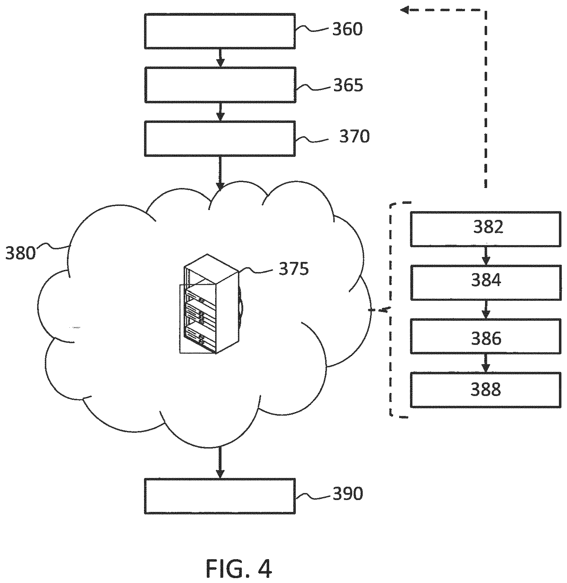

Purely by way of further example, an embodiment will now be described with reference to Figure 4. Figure 4 is a simplified illustration of a process employed by an embodiment which uses a server in a cloud environment.

The exemplary embodiment comprises two main parts: 1. A wearable device collecting sensor data of walking activities; and 2. A server in a cloud-infrastructure employing proposed algorithms for generating a walking aid recommendations.

In step 360, the monitored subject wears a portable monitoring device when undertaking normal daily activities. Here, the device includes a three-axis accelerometer and a barometric air pressure sensor.

In step 365, walking is automatically detected based on the sensor signals. By way of example, this is done based on a known peak detection algorithm.

In step 370, the raw accelerometer and barometer signals of the detected walks are pre-selected to fulfill the requirement of steady state walking with a duration of at least 10 seconds. The pre-selected walking data are transmitted to the server 375 in the cloud environment 380.

Features indicative of walking characteristics are extracted from the transmitted walking data in step 382. Such characteristics include, but are not limited to, walking intensity, regularity, weight-bearing and number of stair walks.

In step 384, the extracted characteristics are aggregated over a specified period (e.g. daily) and stored in a characteristics vector (per subject).

The server employs collaborative filtering and machine learning in step 386 to determine a recommended walking aid based in the extracted characteristics. For example, the algorithm employed by the server of this example is based on the k-Nearest-Neighbors (kNN) algorithm, where subjects are assigned to clusters based on the similarity of their feature vectors. The size k of the clusters may be chosen based on a proportion of the total amount of subjects in the system, for example.

In step 388, the algorithm outputs a percentage of users within the same cluster which use a walking aid and information about the most common type of walking aid used within the cluster.

A walking aid recommendation is then generated based on the output from step 388 and presented to a user in step 390. For example, the walking aid recommendation of this embodiment is in the he format of: "80% of your peers are using a walking aid. 60% of them are using a cane. A cane might be best suited for you."

Furthermore, a feedback loop may be integrated into the system to regularly update information on if and what type of walking aid is used by the user. Firstly, information may be collected during a user registration process using an online form (e.g. "Are you using a walking aid? What type of walking aid are using?"). Subsequently, this information may be updated (e.g. on a monthly basis).

Variations to the above-described embodiments are envisaged whilst still employing the proposed concepts. For example, embodiments may employ stationary monitoring devices (e.g. wall-mounted optical system).

Various other types of sensors may also employed. For example, variations in the type of the wearable sensor (e.g. attached to the skin/body, worn in a pocket or attached to a lanyard) may be used.

Also, deep learning may be used to process the walking activity data and learn abstract characteristics of walking.

Other algorithms/processes for generating recommendations based on characteristics of walking are also envisaged, such as rule-based algorithms, machine learning algorithms and deep learning neural networks.

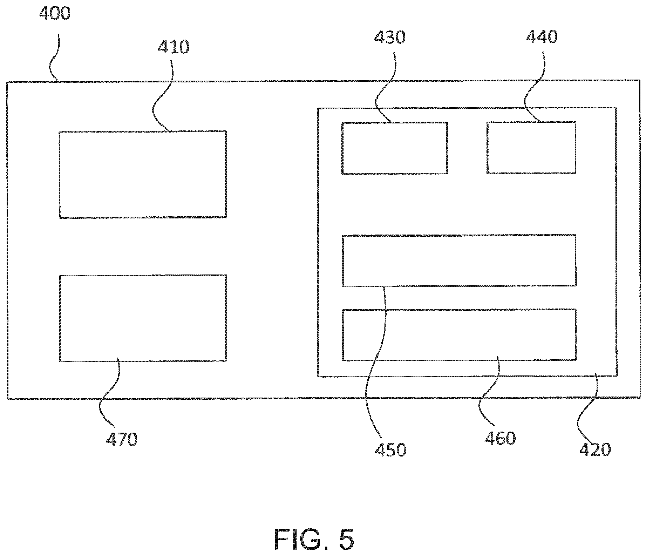

By way of further example, Figure 5 illustrates an example of a computer 400 within which one or more parts of an embodiment may be employed. Various operations discussed above may utilize the capabilities of the computer 400. For example, one or more parts of an ADL monitoring system adapted to monitor walking activity of a subject may be incorporated in any element, module, application, and/or component discussed herein.

The computer 400 includes, but is not limited to, PCs, workstations, laptops, PDAs, palm devices, servers, storages, and the like. Generally, in terms of hardware architecture, the computer 400 may include one or more processors 410, memory 420, and one or more I/O devices 470 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 410 is a hardware device for executing software that can be stored in the memory 420. The processor 410 can be virtually any custom made or commercially available processor, a central processing unit (CPU), a digital signal processor (DSP), or an auxiliary processor among several processors associated with the computer 400, and the processor 410 may be a semiconductor based microprocessor (in the form of a microchip) or a microprocessor.

The memory 420 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 420 may incorporate electronic, magnetic, optical, and/or other types of storage media. Note that the memory 420 can have a distributed architecture, where various components are situated remote from one another, but can be accessed by the processor 410.

The software in the memory 420 may include one or more separate programs, each of which comprises an ordered listing of executable instructions for implementing logical functions. The software in the memory 420 includes a suitable operating system (O/S) 450, compiler 440, source code 430, and one or more applications 460 in accordance with exemplary embodiments. As illustrated, the application 460 comprises numerous functional components for implementing the features and operations of the exemplary embodiments. The application 460 of the computer 400 may represent various applications, computational units, logic, functional units, processes, operations, virtual entities, and/or modules in accordance with exemplary embodiments, but the application 460 is not meant to be a limitation.

The operating system 450 controls the execution of other computer programs, and provides scheduling, input-output control, file and data management, memory management, and communication control and related services. It is contemplated by the inventors that the application 460 for implementing exemplary embodiments may be applicable on all commercially available operating systems.

Application 460 may be a source program, executable program (object code), script, or any other entity comprising a set of instructions to be performed. When a source program, then the program is usually translated via a compiler (such as the compiler 440), assembler, interpreter, or the like, which may or may not be included within the memory 420, so as to operate properly in connection with the O/S 450. Furthermore, the application 460 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, Python, ADA, .NET, and the like.

The I/O devices 470 may include input devices such as, for example but not limited to, a mouse, keyboard, scanner, microphone, camera, etc. Furthermore, the I/O devices 470 may also include output devices, for example but not limited to a printer, display, etc. Finally, the I/O devices 470 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 470 also include components for communicating over various networks, such as the Internet or intranet.

If the computer 400 is a PC, workstation, intelligent device or the like, the software in the memory 420 may further include a basic input output system (BIOS) (omitted for simplicity). The BIOS is a set of essential software routines that initialize and test hardware at startup, start the O/S 450, and support the transfer of data among the hardware devices. The BIOS is stored in some type of read-only-memory, such as ROM, PROM, EPROM, EEPROM or the like, so that the BIOS can be executed when the computer 400 is activated.

When the computer 400 is in operation, the processor 410 is configured to execute software stored within the memory 420, to communicate data to and from the memory 420, and to generally control operations of the computer 400 pursuant to the software. The application 460 and the O/S 450 are read, in whole or in part, by the processor 410, perhaps buffered within the processor 410, and then executed.

When the application 460 is implemented in software it should be noted that the application 460 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 460 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to subjectalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand various embodiments with various modifications are contemplated.

## Claims

1. A system for recommending a walking aid for a subject, wherein the system comprises:
a signal interface (120) adapted to receive activity data representative of walking activity of the subject;
a processing unit (122) adapted to determine a characteristic of walking of the subject based on the activity data, the characteristic of walking of the subject comprising information representing at least one of a pattern of walking of the subject and a parameter of walking of the subject; and
a recommendation unit (124) adapted to determine a walking aid recommendation for the subject based on the characteristic of walking of the subject.

2. The system of claim 1, wherein the activity data representative of walking activity of the subject comprises values at least one of:
a velocity of movement of the subject;
a distance travelled by a body part of the subject;
a rate of acceleration of a body part of the subject;
a location of the subject; and
an air pressure incident on the subject.

3. The system of claim 1 or 2, wherein a parameter of walking of the subject comprises at least one of:
a walking speed of the subject;
a walking intensity of the subject;
a walking stability of the subject;
a walking variability of the subject;
a measure of walking mobility of the subject;
a gait pattern of the subject;
an indoor or outdoor status of the subject;
a walking regularity of the subject;
a walking duration of the subject;
a number of stair visits of the subject; and
a gait abnormality of the subject.

4. The system of any of claims 1 to 3, further comprising a sensor (30) configured to be coupled to a stationary support surface and to generate activity data representative of walking activity of the subject, and preferably wherein the sensor comprises at least one of: an optical sensor; a passive infra-red sensor; and open-close sensor; a power sensor; and a pressure sensor.

5. The system of any of claims 1 to 4 further comprising a sensor (20) configured to be coupled to the subject and to generate activity data representative of walking activity of the subject, and preferably wherein the sensor comprises at least one of: an accelerometer; a gyroscope; a magnetometer; a heart rate sensor; a GPS sensor; a textile-integrated sensor; a movement sensor; a pressure sensor; and a timing device.

6. The system of any of claims 1 to 5, wherein the processing unit (122) is configured to process the activity data with a walking detection algorithm to detect walking activity,
and wherein the processing algorithm is configured to determine a characteristic of walking of the subject based on detected walking activity.

7. The system of claim 6, wherein the walking detection algorithm is configured to detect peaks in a variation of a value with respect to time and to detect walking activity based on detected peaks.

8. The system of any of claims 1 to 4, wherein the recommendation unit (124) comprises:
a classification unit (126) configured to determine a classification of the subject based on the characteristic of walking activity of the subject; and
an analysis unit (128) configured to determine a walking aid recommendation for the subject based on the classification of the subject.

9. The system of claim 8, wherein the classification unit (126) is configured to process the characteristic of walking activity of the subject with at least one of: a machine learning algorithm; a neural network; rule-based algorithm; and comparison algorithm to determine a classification of the subject.

10. The system of claim 8 or 9, wherein the analysis unit (128) is configured to process the classification of the subject with at least one of: a machine learning algorithm; a neural network; rule-based algorithm; and comparison algorithm to determine a walking aid recommendation for the subject.

11. The system of any of claims 8 to 10, wherein the characteristic of walking of the subject comprises information representing a pattern of walking of the subject,
and wherein the classification unit (126) is configured to identify a trend in the pattern of walking of the subject and to determine a classification of the subject based on the identified trend, and preferably wherein the classification unit (126) is configured to detect one or more irregularities in the determined trend, and to determine a classification of the subject based on the detected one or more irregularities in the determined trend.

12. The system of any of claim 11, wherein the classification unit (126) is configured to identify whether there are one or more irregularities in the determined trend based on at least one of: a comparison of the determined trend with a threshold value; a comparison of a value of a characteristic of walking of the subject with an average value; and a comparison of the determined trend with a predetermined trend, and preferably wherein the threshold value is determined based on at least one of:
one or more previously obtained values of a property of at least one of: a pattern of walking of the subject and a parameter of walking of the subject; and
one or more previously determined changes in values of a property of at least one of: pattern of walking of the subject and a parameter of walking of the subject.

13. The system of any of claims 1 to 12, wherein the walking aid recommendation for the subject comprises: a description of a walking aid; and a numerical value associated with the walking aid.

14. A method for recommending a walking aid for a subject, wherein the system comprises:
obtaining activity data representative of walking activity of the subject;
determining a characteristic of walking of the subject based on the activity data, the characteristic of walking of the subject comprising information representing at least one of a pattern of walking of the subject and a parameter of walking of the subject; and
determining a walking aid recommendation for the subject based on the characteristic of walking of the subject.

15. A computer program product for recommending a walking aid for a subject, wherein the computer program product comprises a computer-readable storage medium having computer-readable program code embodied therewith, the computer-readable program code configured to perform all of the steps of claim 14.
